(19) Europäisches Patentamt
European Patent Office
Office européen des brevets

(11) EP 4 129 988 A1

(12) **EUROPEAN PATENT APPLICATION**
published in accordance with Art. 153(4) EPC

(43) Date of publication:
**08.02.2023 Bulletin 2023/06**

(21) Application number: **21775104.9**

(22) Date of filing: **19.03.2021**

(51) International Patent Classification (IPC):
***C07D 257/06*** *(1974.07)* ***A01P 13/00*** *(2006.01)*
***A01N 43/713*** *(1985.01)* ***A01N 43/84*** *(1980.01)*

(52) Cooperative Patent Classification (CPC):
**A01N 43/713; A01N 43/84; A01P 13/00; C07D 257/06**

(86) International application number:
**PCT/JP2021/011323**

(87) International publication number:
**WO 2021/193410 (30.09.2021 Gazette 2021/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.03.2020 JP 2020051359**

(71) Applicant: **Nippon Soda Co., Ltd.**
**Tokyo 100-8165 (JP)**

(72) Inventors:
- **IKEDA Yoji**
  **Odawara-shi, Kanagawa 250-0280 (JP)**
- **TAKI Yukina**
  **Odawara-shi, Kanagawa 250-0280 (JP)**
- **INAGAKI Jun**
  **Odawara-shi, Kanagawa 250-0280 (JP)**

(74) Representative: **Wibbelmann, Jobst**
**Wuesthoff & Wuesthoff**
**Patentanwälte PartG mbB**
**Schweigerstrasse 2**
**81541 München (DE)**

(54) **BENZAMIDE COMPOUND AND HERBICIDE**

(57) A compound represented by a formula (I) or a salt thereof; and a herbicide containing at least one selected from the group consisting of the compound and a salt thereof as an active ingredient.

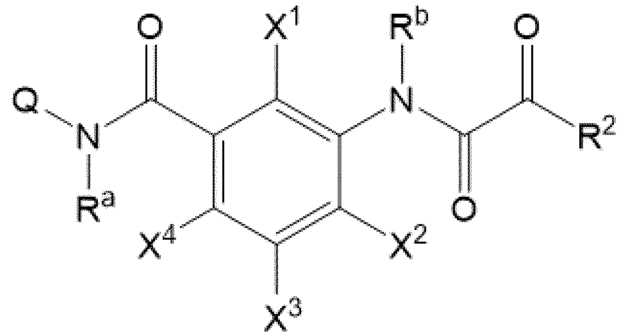

(I)

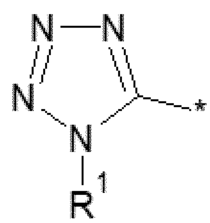

(Q1)

wherein $R^a$ and $R^b$ each independently represent a hydrogen atom, a substituted or unsubstituted $C_{1-6}$ alkyl group, or the like; $X^1$ and $X^2$ each independently represent a hydrogen atom, a substituted or unsubstituted $C_{1-6}$ alkyl group, or the like; $X^3$ represents a hydrogen atom, a substituted or unsubstituted $C_{1-6}$ alkyl group, or a halogeno group; $X^4$ represents a hydrogen atom or a halogeno group; $R^2$ represents a substituted or unsubstituted $C_{1-6}$ alkyl group, or the like; Q represents a group represented by a formula (Q1), or the like; and in the formula (Q1), an asterisk (*) indicates a bonding position with an $R^a$-substituted nitrogen atom, and each $R^1$ independently represents a hydrogen atom or a substituted or unsubstituted $C_{1-6}$ alkyl group.

EP 4 129 988 A1

## Description

BACKGROUND OF THE INVENTION

Field of the Invention

The present invention relates to a novel benzamide compound and a herbicide containing the same as an active ingredient.

The present application claims priority on Japanese Patent Application No. 2020-051359, filed in Japan on March 23, 2020, the content of which is incorporated herein by reference.

Description of the Related Art

In the cultivation of agricultural and horticultural crops, herbicides may be used for controlling weeds. Various compounds have been proposed so far as active ingredients of herbicides.

For example, Patent Document 1 discloses a benzamide compound represented by a formula (1).

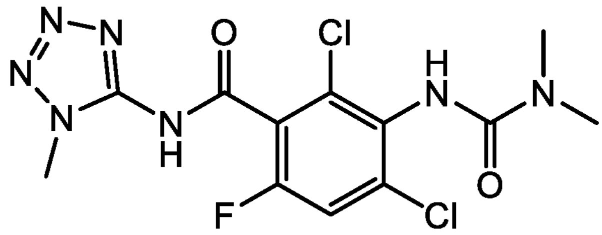

(1)

Patent Document 2 discloses a benzamide compound represented by a formula (2).

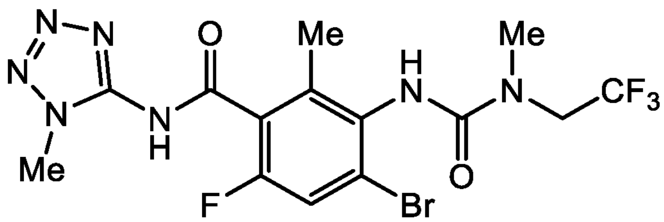

(2)

PRIOR ART DOCUMENTS

[Patent Documents]

[Patent Document 1] WO2017 / 102275A
[Patent Document 2] WO2019 / 105995A

SUMMARY OF THE INVENTION

Problems to be Solved by the Invention

Herbicides are required not only to have an excellent weed control effect, but also to have less phytotoxicity to crops, to be less likely to remain in the environment, and not to pollute the environment.

An object of the present invention is to provide a novel benzamide compound useful as an active ingredient of a herbicide, which has a reliable weed control effect even at a low dose, has less phytotoxicity to crops, and is highly safe for the environment; and a herbicide.

Means for Solving the Problem

As a result of intensive studies in order to achieve the above object, the present invention including the following embodiments has been completed.

[1] A compound represented by a formula (I) or a salt thereof:

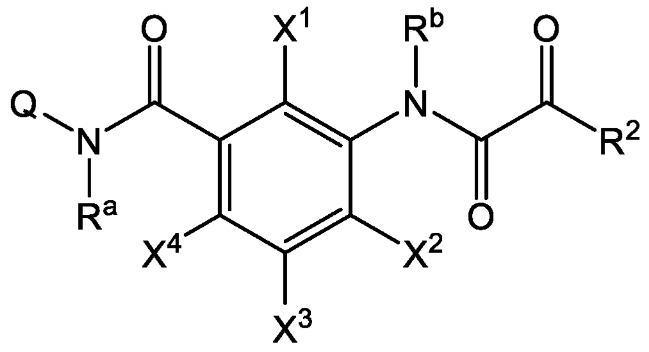

(I)

(In the formula (I),

$R^a$ represents a hydrogen atom, a substituted or unsubstituted $C_{1-6}$ alkyl group, a substituted or unsubstituted $C_{1-6}$ alkylcarbonyl group, a substituted or unsubstituted $C_{1-6}$ alkoxycarbonyl group, or a substituted or unsubstituted $C_{6-10}$ arylcarbonyl group,
$R^b$ represents a hydrogen atom, a substituted or unsubstituted $C_{1-6}$ alkyl group, a substituted or unsubstituted $C_{1-6}$ alkylcarbonyl group, a substituted or unsubstituted $C_{1-6}$ alkoxycarbonyl group, or a substituted or unsubstituted $C_{6-10}$ arylcarbonyl group,
$X^1$ represents a hydrogen atom, a substituted or unsubstituted $C_{1-6}$ alkyl group, a substituted or unsubstituted $C_{1-6}$ alkylthio group, a substituted or unsubstituted $C_{1-6}$ alkylsulfinyl group, a substituted or unsubstituted $C_{1-6}$ alkylsulfonyl group, or a halogeno group,
$X^2$ represents a hydrogen atom, a substituted or unsubstituted $C_{1-6}$ alkyl group, a substituted or unsubstituted $C_{1-6}$ alkylthio group, a substituted or unsubstituted $C_{1-6}$ alkylsulfinyl group, a substituted or unsubstituted $C_{1-6}$ alkylsulfonyl group, or a halogeno group,
$X^3$ represents a hydrogen atom, a substituted or unsubstituted $C_{1-6}$ alkyl group, or a halogeno group,
$X^4$ represents a hydrogen atom or a halogeno group,
$R^2$ represents a substituted or unsubstituted $C_{1-6}$ alkyl group, a substituted or unsubstituted $C_{1-6}$ alkoxy group, or a group represented by a formula: $NR^{2a}R^{2b}$,
$R^{2a}$ represents a hydrogen atom, a substituted or unsubstituted $C_{1-6}$ alkyl group, or a substituted or unsubstituted $C_{1-6}$ alkoxy group,
$R^{2b}$ represents a hydrogen atom or a substituted or unsubstituted $C_{1-6}$ alkyl group,
$R^{2a}$ and $R^{2b}$ may form a divalent organic group together, and
Q represents a group represented by a formula (Q1), formula (Q2), formula (Q3) or formula (Q4),

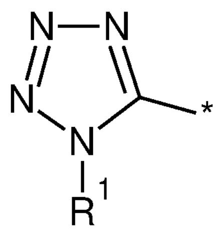

(Q1)

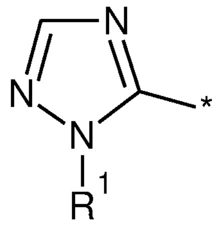

(Q2)

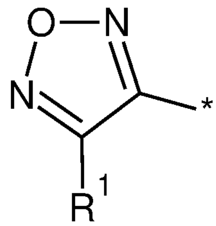

(Q3)

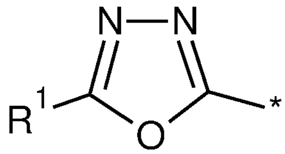

(Q4)

In the formula (Q1), formula (Q2), formula (Q3) and formula (Q4),

an asterisk (*) indicates a bonding position with an $R^a$-substituted nitrogen atom, and
each $R^1$ independently represents a hydrogen atom or a substituted or unsubstituted $C_{1-6}$ alkyl group.)

[2] The compound according to [1] or a salt thereof, wherein $R^2$ is a group represented by the formula: $NR^{2a}R^{2b}$ (the above $R^{2a}$ and $R^{2b}$ are as defined by the formula (I) in [1].)
[3] A herbicide containing at least one selected from the group consisting of the compound according to either [1] or [2] and a salt thereof as an active ingredient.

Effects of the Invention

Since the benzamide compound of the present invention has a reliable weed control effect even at a low dose, has less phytotoxicity to crops, and is highly safe for the environment, it is useful as an active ingredient of a herbicide. The herbicide of the present invention can be safely used for controlling weeds in the cultivation of agricultural and horticultural crops.

DETAILED DESCRIPTION OF THE INVENTION

The benzamide compound of the present invention is a compound represented by the formula (I) (sometimes referred to as compound (I)) and a salt of the compound (I). The benzamide compound of the present invention also includes hydrates, various solvates, polymorphs of crystals and the like. The benzamide compound of the present invention may have stereoisomers based on asymmetric carbons, double bonds and the like, and tautomers. All such isomers and mixtures thereof are within the technical scope of the present invention.
The term "unsubstituted" used in the present specification means that it is composed only of a group which becomes a mother nucleus. When it is described only by the name of the group which becomes the mother nucleus without being described as "substituted", it means "unsubstituted" unless otherwise stated.
On the other hand, the term "substituted" means that any hydrogen atom of a group which becomes a mother nucleus is substituted with a group (substituent) having the same or different structure as that of the mother nucleus. Therefore, a "substituent" is another group bonded to a group which becomes a mother nucleus. The number of substituents may be one, or two or more. The two or more substituents may be the same or different.
The terms "$C_{1-6}$" and the like mean that the number of carbon atoms in the group which becomes a mother nucleus is 1 to 6, and so on. The number of carbon atoms does not include the number of carbon atoms present in the

substituent. For example, a butyl group having an ethoxy group as a substituent is classified into a C2 alkoxy C4 alkyl group.

A "substituent" is not particularly limited as long as it is chemically acceptable and has the effects of the present invention. Hereinafter, groups which can be a "substituent" are exemplified.

A$C_{1-6}$ alkyl group such as a methyl group, an ethyl group, an n-propyl group, an i-propyl group, an n-butyl group, an s-butyl group, an i-butyl group, a t-butyl group, an n-pentyl group and an n-hexyl group;

a $C_{2-6}$ alkenyl group such as a vinyl group, a 1-propenyl group, a 2-propenyl group (allyl group), a 1-butenyl group, a 2-butenyl group, a 3-butenyl group, a 1-methyl-2-propenyl group and a 2-methyl-2-propenyl group;
a $C_{2-6}$ alkynyl group such as an ethynyl group, a 1-propynyl group, a 2-propynyl group, a 1-butynyl group, a 2-butynyl group, a 3-butynyl group and a 1-methyl-2-propynyl group;
a $C_{3-6}$ cycloalkyl group such as a cyclopropyl group, a cyclobutyl group, a cyclopentyl group and a cyclohexyl group;
a $C_{6-10}$ aryl group such as a phenyl group and a naphthyl group;
a $C_{6-10}$ aryl $C_{1-6}$ alkyl group such as a benzyl group and a phenethyl group;
a 3- to 6-membered heterocyclyl group;
a 3- to 6-membered heterocyclyl $C_{1-6}$ alkyl group;
a hydroxyl group;
a $C_{1-6}$ alkoxy group such as a methoxy group, an ethoxy group, an n-propoxy group, an i-propoxy group, an n-butoxy group, an s-butoxy group, an i-butoxy group and a t-butoxy group;
a $C_{2-6}$ alkenyloxy group such as a vinyloxy group, an allyloxy group, a propenyloxy group and a butenyloxy group;
a $C_{2-6}$ alkynyloxy group such as an ethynyloxy group and a propargyloxy group;
a $C_{6-10}$ aryloxy group such as a phenoxy group and a naphthoxy group;
a $C_{6-10}$ aryl $C_{1-6}$ alkoxy group such as a benzyloxy group and a phenethyloxy group;
a 5- to 6-membered heteroaryloxy group such as a thiazolyloxy group and a pyridyloxy group;
a 5- to 6-membered heteroaryl $C_{1-6}$ alkyloxy group such as a thiazolylmethyloxy group and a pyridylmethyloxy group;
a formyl group;
a $C_{1-6}$ alkylcarbonyl group such as an acetyl group and a propionyl group;
a formyloxy group;
a $C_{1-6}$ alkylcarbonyloxy group such as an acetyloxy group and a propionyloxy group;
a $C_{6-10}$ arylcarbonyl group such as a benzoyl group;
a $C_{1-6}$ alkoxycarbonyl group such as a methoxycarbonyl group, an ethoxycarbonyl group, an n-propoxycarbonyl group, an i-propoxycarbonyl group, an n-butoxycarbonyl group and a t-butoxycarbonyl group;
a $C_{1-6}$ alkoxycarbonyloxy group such as a methoxycarbonyloxy group, an ethoxycarbonyloxy group, an n-propoxycarbonyloxy group, an i-propoxycarbonyloxy group, an n-butoxycarbonyloxy group and a t-butoxycarbonyloxy group;
a carboxyl group;
a halogeno group such as a fluoro group, a chloro group, a bromo group and an iodo group;
a $C_{1-6}$ haloalkyl group such as a chloromethyl group, a chloroethyl group, a trifluoromethyl group, a 1,2-dichloro-n-propyl group, a 1-fluoro-n-butyl group and a 2,2,2-trifluoroethyl group;
a $C_{2-6}$ haloalkenyl group such as a 2-chloro-1-propenyl group and a 2-fluoro-1-butenyl group;
a $C_{2-6}$ haloalkynyl group such as a 4,4-dichloro-1-butynyl group, a 4-fluoro-1-pentynyl group and a 5-bromo-2-pentynyl group;
a $C_{1-6}$ haloalkoxy group such as a trifluoromethoxy group, a 2-chloro-n-propoxy group and a 2,3-dichlorobutoxy group;
a $C_{2-6}$ haloalkenyloxy group such as a 2-chloropropenyloxy group and a 3-bromobutenyloxy group;
a $C_{1-6}$ haloalkylcarbonyl group such as a chloroacetyl group, a trifluoroacetyl group and a trichloroacetyl group;
an amino group;
a $C_{1-6}$ alkyl-substituted amino group such as a methylamino group, a dimethylamino group and a diethylamino group;
a $C_{6-10}$ arylamino group such as an anilino group and a naphthylamino group;
a $C_{6-10}$ aryl $C_{1-6}$ alkylamino group such as a benzylamino group and a phenethylamino group;
a formylamino group;
a $C_{1-6}$ alkylcarbonylamino group such as an acetylamino group, a propanoylamino group, a butyrylamino group and an i-propylcarbonylamino group;
a $C_{1-6}$ alkoxycarbonylamino group such as a methoxycarbonylamino group, an ethoxycarbonylamino group, an n-propoxycarbonylamino group and an i-propoxycarbonylamino group;
an unsubstituted or substituted aminocarbonyl group such as an aminocarbonyl group, a dimethylaminocarbonyl group, a phenylaminocarbonyl group and an N-phenyl-N-methylaminocarbonyl group;
an imino $C_{1-6}$ alkyl group such as an iminomethyl group, a (1-imino)ethyl group and a (1-imino)-n-propyl group;

a substituted or unsubstituted N-hydroxyimino $C_{1-6}$ alkyl group such as an N-hydroxy-iminomethyl group, a (1-(N-hydroxy)-imino)ethyl group, a (1-(N-hydroxy)-imino)propyl group, an N-methoxy-iminomethyl group, and a (1-(N-methoxy)-imino)ethyl group;
an aminocarbonyloxy group;
a $C_{1-6}$ alkyl-substituted aminocarbonyloxy group such as an ethylaminocarbonyloxy group and a dimethylaminocarbonyloxy group;
a mercapto group;
a $C_{1-6}$ alkylthio group such as a methylthio group, an ethylthio group, an n-propylthio group, an i-propylthio group, an n-butylthio group, an i-butylthio group, an s-butylthio group and a t-butylthio group;
a $C_{1-6}$ haloalkylthio group such as a trifluoromethylthio group and a 2,2,2-trifluoroethylthio group;
a $C_{6-10}$ arylthio group such as a phenylthio group and a naphthylthio group;
a 5- to 6-membered heteroarylthio group such as a thiazolylthio group and a pyridylthio group;
a $C_{1-6}$ alkylsulfinyl group such as a methylsulfinyl group, an ethylsulfinyl group and a t-butylsulfinyl group;
a $C_{1-6}$ haloalkylsulfinyl group such as a trifluoromethyl sulfinyl group and a 2,2,2-trifluoroethylsulfinyl group;
a $C_{6-10}$ arylsulfinyl group such as a phenylsulfinyl group;
a 5- to 6-membered heteroarylsulfinyl group such as a thiazolylsulfinyl group and a pyridylsulfinyl group;
a $C_{1-6}$ alkylsulfonyl group such as a methylsulfonyl group, an ethylsulfonyl group and a t-butylsulfonyl group;
a $C_{1-6}$ haloalkylsulfonyl group such as a trifluoromethylsulfonyl group and a 2,2,2-trifluoroethylsulfonyl group;
a $C_{6-10}$ arylsulfonyl group such as a phenylsulfonyl group;
a 5- to 6-membered heteroarylsulfonyl group such as a thiazolylsulfonyl group and a pyridylsulfonyl group;
a $C_{1-6}$ alkylsulfonyloxy group such as a methylsulfonyloxy group, an ethylsulfonyloxy group and a t-butylsulfonyloxy group;
a $C_{1-6}$ haloalkylsulfonyloxy group such as a trifluoromethylsulfonyloxy group and a 2,2,2-trifluoroethylsulfonyloxy group;
a tri $C_{1-6}$ alkyl-substituted silyl group such as a trimethylsilyl group, a triethylsilyl group and a t-butyldimethylsilyl group;
a tri $C_{6-10}$ aryl-substituted silyl group such as a triphenylsilyl group;
a pentafluorosulfanyl group;
a cyano group; a nitro group.

Further, in these "substituents", any hydrogen atom in the substituent may be substituted with a group having a different structure. Examples of the "substituent" in this case include a $C_{1-6}$ alkyl group, a $C_{1-6}$ haloalkyl group, a $C_{1-6}$ alkoxy group, a $C_{1-6}$ haloalkoxy group, a halogeno group, a cyano group and a nitro group.

Further, the above-described "3- to 6-membered heterocyclyl group" includes 1 to 4 hetero atoms selected from the group consisting of a nitrogen atom, an oxygen atom and a sulfur atom as constituent atoms of the ring. The heterocyclyl group may be either monocyclic or polycyclic. As long as the polycyclic heterocyclyl group includes at least one heterocyclic ring, the remaining ring may be any of a saturated alicyclic ring, an unsaturated alicyclic ring or an aromatic ring. Examples of the "3- to 6-membered heterocyclyl group" include a 3- to 6-membered saturated heterocyclyl group, a 5- to 6-membered heteroaryl group and a 5- to 6-membered partially unsaturated heterocyclyl group.

Examples of the 3- to 6-membered saturated heterocyclyl group include an aziridinyl group, an epoxy group, a pyrrolidinyl group, a tetrahydrofuranyl group, a thiazolidinyl group, a piperidyl group, a piperazinyl group, a morpholinyl group, a dioxolanyl group and a dioxanyl group.

Examples of the 5-membered heteroaryl group include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group and a tetrazolyl group.

Examples of the 6-membered heteroaryl group include a pyridyl group, a pyrazinyl group, a pyrimidinyl group, a pyridazinyl group and a triazinyl group.

[Q]

In the formula (I), Q represents a group represented by the formula (Q1), formula (Q2), formula (Q3) or formula (Q4).

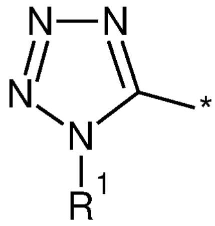

(Q1)

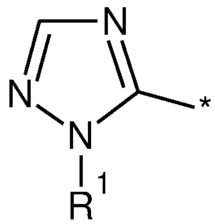

(Q2)

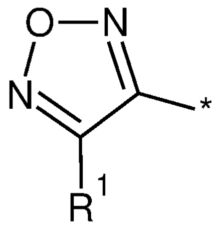

(Q3)

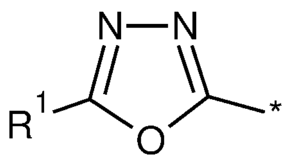

(Q4)

In the formula (Q1), formula (Q2), formula (Q3) and formula (Q4), an asterisk (*) indicates a bonding position with an $R^a$-substituted nitrogen atom.

In the formula (Q1), formula (Q2), formula (Q3) and formula (Q4), each $R^1$ independently represents a hydrogen atom or a substituted or unsubstituted $C_{1-6}$ alkyl group, preferably a methyl group.

Q is preferably a group represented by the formula (Q1), formula (Q2) or formula (Q4), and more preferably a group represented by the formula (Q1).

The "$C_{1-6}$ alkyl group" represented by $R^1$ may be linear or branched. Examples of the "$C_{1-6}$ alkyl group" include a methyl group, an ethyl group, an n-propyl group, an n-butyl group, an n-pentyl group, an n-hexyl group, an i-propyl group, an i-butyl group, an s-butyl group, a t-butyl group, an i-pentyl group, a neopentyl group, a 2-methylbutyl group and an i-hexyl group.

Examples of the substituent on the "$C_{1-6}$ alkyl group" represented by $R^1$ include a halogeno group such as a fluoro group, a chloro group, a bromo group and an iodo group; a hydroxyl group; a $C_{1-6}$ alkoxy group such as a methoxy group, an ethoxy group, an n-propoxy group, an i-propoxy group, an n-butoxy group, an s-butoxy group, an i-butoxy group and a t-butoxy group; a $C_{1-6}$ haloalkoxy group such as a 2-chloro-n-propoxy group, a 2,3-dichlorobutoxy group and a trifluoromethoxy group; a $C_{6-10}$ aryl group such as a phenyl group and a naphthyl group; a $C_{6-10}$ aryl group substituted with a halogeno group, $C_{1-6}$ haloalkyl group or $C_{1-6}$ haloalkoxy group, such as a 4-chlorophenyl group, a 4-trifluoromethylphenyl group and a 4-trifluoromethoxyphenyl group; or a cyano group.

[$R^a$, $R^b$]

In the formula (I), $R^a$ represents a hydrogen atom, a substituted or unsubstituted $C_{1-6}$ alkyl group, a substituted or unsubstituted $C_{1-6}$ alkylcarbonyl group, a substituted or unsubstituted $C_{1-6}$ alkoxycarbonyl group, or a substituted or unsubstituted $C_{6-10}$ arylcarbonyl group.

In the formula (I), $R^b$ represents a hydrogen atom, a substituted or unsubstituted $C_{1-6}$ alkyl group, a substituted

or unsubstituted $C_{1-6}$ alkylcarbonyl group, a substituted or unsubstituted $C_{1-6}$ alkoxycarbonyl group, or a substituted or unsubstituted $C_{6-10}$ arylcarbonyl group.

Specific examples of the "$C_{1-6}$ alkyl group" represented by $R^a$ and $R^b$ and specific examples of the substituent on the "$C_{1-6}$ alkyl group" include the same as those exemplified for $R^1$.

Examples of the "$C_{1-6}$ alkylcarbonyl group" represented by $R^a$ and $R^b$ include an acetyl group, a propionyl group, a butyroyl group, a pentanoyl group and a hexanoyl group.

Examples of the "$C_{1-6}$ alkoxycarbonyl group" represented by $R^a$ and $R^b$ include a methoxycarbonyl group, an ethoxycarbonyl group, an n-propoxycarbonyl group, an i-propoxycarbonyl group, an n-butoxycarbonyl group, an i-butoxycarbonyl group, an s-butoxycarbonyl group, a t-butoxycarbonyl group, an n-pentyloxycarbonyl group and an n-hexyloxycarbonyl group.

Examples of the "$C_{6-10}$ arylcarbonyl group" represented by $R^a$ and $R^b$ include an arylcarbonyl group such as a benzoyl group, a naphthylcarbonyl group and an anthranilcarbonyl group.

Examples of the substituent on the "$C_{1-6}$ alkylcarbonyl group" or "$C_{1-6}$ alkoxycarbonyl group" represented by $R^a$ and $R^b$ include a halogeno group such as a fluoro group, a chloro group, a bromo group and an iodo group; a hydroxyl group; a $C_{1-6}$ alkoxy group such as a methoxy group, an ethoxy group, an n-propoxy group, an i-propoxy group, an n-butoxy group, an s-butoxy group, an i-butoxy group and a t-butoxy group; a $C_{1-6}$ haloalkoxy group such as a 2-chloro-n-propoxy group, a 2,3-dichlorobutoxy group and a trifluoromethoxy group; a $C_{6-10}$ aryl group such as a phenyl group and a naphthyl group;

a $C_{6-10}$ aryl group substituted with a halogeno group, $C_{1-6}$ haloalkyl group or $C_{1-6}$ haloalkoxy group, such as a 4-chlorophenyl group, a 4-trifluoromethylphenyl group and a 4-trifluoromethoxyphenyl group; or a cyano group.

As the substituent on the "$C_{6-10}$ arylcarbonyl group" represented by $R^a$ and $R^b$, a halogeno group such as a fluoro group, a chloro group, a bromo group and an iodo group; a $C_{1-6}$ alkyl group such as a methyl group, an ethyl group, an n-propyl group, an i-propyl group, an n-butyl group, an s-butyl group, an i-butyl group, a t-butyl group, an n-pentyl group and an n-hexyl group; a $C_{1-6}$ haloalkyl group such as a chloromethyl group, a chloroethyl group, a trifluoromethyl group, a 1,2-dichloro-n-propyl group and a 1-fluoro-n-butyl group; a hydroxyl group; a $C_{1-6}$ alkoxy group such as a methoxy group, an ethoxy group, an n-propoxy group, an i-propoxy group, an n-butoxy group, an s-butoxy group, an i-butoxy group and a t-butoxy group; a $C_{1-6}$ haloalkoxy group such as a 2-chloro-n-propoxy group, a 2,3-dichlorobutoxy group and a trifluoromethoxy group; or a cyano group; is preferable.

As $R^a$ and $R^b$, a hydrogen atom is preferable.

[$X^1$, $X^2$]

In the formula (I), $X^1$ represents a hydrogen atom, a substituted or unsubstituted $C_{1-6}$ alkyl group, a substituted or unsubstituted $C_{1-6}$ alkylthio group, a substituted or unsubstituted $C_{1-6}$ alkylsulfinyl group, a substituted or unsubstituted $C_{1-6}$ alkylsulfonyl group, or a halogeno group.

In the formula (I), $X^2$ represents a hydrogen atom, a substituted or unsubstituted $C_{1-6}$ alkyl group, a substituted or unsubstituted $C_{1-6}$ alkylthio group, a substituted or unsubstituted $C_{1-6}$ alkylsulfinyl group, a substituted or unsubstituted $C_{1-6}$ alkylsulfonyl group, or a halogeno group.

Specific examples of the "$C_{1-6}$ alkyl group" represented by $X^1$ and $X^2$ and specific examples of the substituent on the "$C_{1-6}$ alkyl group" include the same as those exemplified for $R^1$.

Examples of the "$C_{1-6}$ alkylthio group" represented by $X^1$ and $X^2$ include a methylthio group, an ethylthio group, an n-propylthio group, an i-propylthio group, an n-butylthio group, an i-butylthio group, an s-butylthio group and a t-butylthio group.

Examples of the "$C_{1-6}$ alkylsulfinyl group" represented by $X^1$ and $X^2$ include a methylsulfinyl group, an ethylsulfinyl group and a t-butylsulfinyl group.

Examples of the "$C_{1-6}$ alkylsulfonyl group" represented by $X^1$ and $X^2$ include a methylsulfonyl group, an ethylsulfonyl group and a t-butylsulfonyl group.

Examples of the "halogeno group" represented by $X^1$ and $X^2$ include a fluoro group, a chloro group, a bromo group and an iodo group.

Examples of the substituent on the "$C_{1-6}$ alkylthio group", "$C_{1-6}$ alkylsulfinyl group" or "$C_{1-6}$ alkylsulfonyl group" represented by $X^1$ and $X^2$ include a halogeno group such as a fluoro group, a chloro group, a bromo group and an iodo group; a hydroxyl group; a $C_{1-6}$ alkoxy group such as a methoxy group, an ethoxy group, an n-propoxy group, an i-propoxy group, an n-butoxy group, an s-butoxy group, an i-butoxy group and a t-butoxy group; a $C_{1-6}$ haloalkoxy group such as a 2-chloro-n-propoxy group, a 2,3-dichlorobutoxy group and a trifluoromethoxy group; a $C_{6-10}$ aryl group such as a phenyl group and a naphthyl group; a $C_{6-10}$ aryl group substituted with a halogeno group, $C_{1-6}$ haloalkyl group or $C_{1-6}$ haloalkoxy group, such as a 4-chlorophenyl group, a 4-trifluoromethylphenyl group and a 4-trifluoromethoxyphenyl group; or a cyano group.

As $X^1$ and $X^2$, a hydrogen atom, a substituted or unsubstituted $C_{1-6}$ alkyl group and a halogeno group are

preferable, and a substituted or unsubstituted $C_{1-6}$ alkyl group and a halogeno group are more preferable.

[$X^3$]

In the formula (I), $X^3$ represents a hydrogen atom, a substituted or unsubstituted $C_{1-6}$ alkyl group, or a halogeno group.
Specific examples of the "$C_{1-6}$ alkyl group" represented by $X^3$ and specific examples of the substituent on the "$C_{1-6}$ alkyl group" include the same as those exemplified for $R^1$.
Specific examples of the "halogeno group" represented by $X^3$ include the same as those exemplified for $X^1$.
As $X^3$, a hydrogen atom is preferable.

[$X^4$]

In the formula (I), $X^4$ represents a hydrogen atom or a halogeno group.
Specific examples of the "halogeno group" represented by $X^4$ include the same as those exemplified for $X^1$.
As $X^4$, a halogeno group is preferable.

[R2]

In the formula (I), $R^2$ represents a substituted or unsubstituted $C_{1-6}$ alkyl group, a substituted or unsubstituted $C_{1-6}$ alkoxy group, or a group represented by the formula: $NR^{2a}R^{2b}$.
Specific examples of the "$C_{1-6}$ alkyl group" represented by $R^2$ and specific examples of the substituent on the "$C_{1-6}$ alkyl group" include the same as those exemplified for $R^1$.
Examples of the "$C_{1-6}$ alkoxy group" represented by $R^2$ include a methoxy group, an ethoxy group, an n-propoxy group, an i-propoxy group, an n-butoxy group, an s-butoxy group, an i-butoxy group and a t-butoxy group.
Examples of the substituent on the "$C_{1-6}$ alkoxy group" represented by $R^2$ include a halogeno group such as a fluoro group, a chloro group, a bromo group and an iodo group; a hydroxyl group; a $C_{1-6}$ alkoxy group such as a methoxy group, an ethoxy group, an n-propoxy group, an i-propoxy group, an n-butoxy group, an s-butoxy group, an i-butoxy group and a t-butoxy group; a $C_{1-6}$ haloalkoxy group such as a 2-chloro-n-propoxy group, a 2,3-dichlorobutoxy group and a trifluoromethoxy group; a $C_{6-10}$ aryl group such as a phenyl group and a naphthyl group; a $C_{6-10}$ aryl group substituted with a halogeno group, $C_{1-6}$ haloalkyl group or $C_{1-6}$ haloalkoxy group, such as a 4-chlorophenyl group, a 4-trifluoromethylphenyl group and a 4-trifluoromethoxyphenyl group; or a cyano group.
In the "group represented by the formula: $NR^{2a}R^{2b}$" represented by $R^2$, $R^{2a}$ represents a hydrogen atom, a substituted or unsubstituted $C_{1-6}$ alkyl group, or a substituted or unsubstituted $C_{1-6}$ alkoxy group, and $R^{2b}$ represents a hydrogen atom or a substituted or unsubstituted $C_{1-6}$ alkyl group. Here, $R^{2a}$ and $R^{2b}$ may form a divalent organic group together.
Specific examples of the "$C_{1-6}$ alkyl group" represented by $R^{2a}$ and $R^{2b}$ and specific examples of the substituent on the "$C_{1-6}$ alkyl group" include the same as those exemplified for $R^1$.
Specific examples of the "$C_{1-6}$ alkoxy group" represented by $R^{2a}$ and specific examples of the substituent on the "$C_{1-6}$ alkoxy group" include the same as those exemplified for $R^2$.
Examples of the "divalent organic group" represented by $R^{2a}$ and $R^{2b}$ include a substituted or unsubstituted $C_{2-5}$ alkylene group and a substituted or unsubstituted $C_{1-2}$ alkyleneoxy $C_{1-2}$ alkylene group.
Examples of the "$C_{2-5}$ alkylene group" represented by $R^{2a}$ and $R^{2b}$ include a dimethylene group, a trimethylene group, a tetramethylene group and a pentamethylene group.
Examples of the "$C_{1-2}$ alkyleneoxy $C_{1-2}$ alkylene group" represented by $R^{2a}$ and $R^{2b}$ include a methyleneoxymethylene group ($-CH_2-O-CH_2-$), a methyleneoxydimethylene group ($-CH_2-O-CH_2CH_2-$), a dimethyleneoxymethylene group ($-CH_2CH_2-O-CH_2-$) and a dimethyleneoxydimethylene group ($-CH_2CH_2-O-CH_2CH_2-$).
Examples of the substituent on the "$C_{2-4}$ alkylene group" or "$C_{1-2}$ alkyleneoxy $C_{1-2}$ alkylene group" represented by $R^{2a}$ and $R^{2b}$ include a halogeno group such as a fluoro group, a chloro group, a bromo group and an iodo group; a $C_{1-6}$ alkyl group such as a methyl group, an ethyl group, an n-propyl group, an i-propyl group, an n-butyl group, an s-butyl group, an i-butyl group and a t-butyl group; a $C_{1-6}$ haloalkyl group such as a chloromethyl group, a chloroethyl group, a trifluoromethyl group, a 1,2-dichloro-n-propyl group and a 1-fluoro-n-butyl group; a hydroxyl group; a $C_{1-6}$ alkoxy group such as a methoxy group, an ethoxy group, an n-propoxy group, an i-propoxy group, an n-butoxy group, an s-butoxy group, an i-butoxy group and a t-butoxy group; a $C_{1-6}$ haloalkoxy group such as a 2-chloro-n-propoxy group, a 2,3-dichlorobutoxy group and a trifluoromethoxy group; or a cyano group.
As $R^2$, a group represented by the formula: $NR^{2a}R^{2b}$ is preferable.
When $R^2$ is a group represented by the formula: $NR^{2a}R^{2b}$, $R^{2a}$ is preferably a substituted or unsubstituted $C_{1-6}$ alkyl group or a substituted or unsubstituted $C_{1-6}$ alkoxy group, and more preferably a substituted or unsubstituted $C_{1-6}$

alkyl group.

When $R^2$ is a group represented by the formula: $NR^{2a}R^{2b}$, $R^{2b}$ is preferably a substituted or unsubstituted $C_{1-6}$ alkyl group.

[Salt]

Examples of a salt of the compound (I) include salts of alkali metals such as lithium, sodium and potassium; salts of alkaline earth metals such as calcium and magnesium; salts of transition metals such as iron and copper; ammonium salts; and salts of organic bases such as triethylamine, tributylamine, pyridine and hydrazine.

The structure of the benzamide compound of the present invention can be determined by NMR spectra, IR spectra, MS spectra and the like.

[Method for producing benzamide compound]

The benzamide compound of the present invention is not particularly limited by the production method thereof. Further, the salt of compound (I) can be obtained from the compound (I) by a known method. As a method for producing the benzamide compound of the present invention, for example, in addition to the method described in Examples and the like, a known reaction as described in the reaction scheme, Examples and the like shown below can be used.

(Reaction scheme 1)

An outline of a method for preparing the compound of the formula (I) is shown in the following reaction scheme 1.

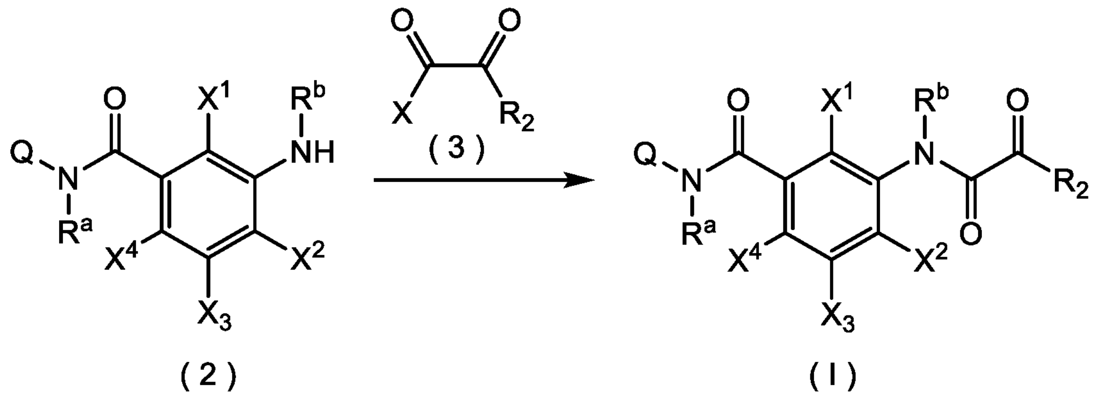

The symbols in the formula (I) have the same meanings as those defined above. The symbols in the formula (2) have the same meanings as those defined in the formula (I). $R^2$ in the formula (3) has the same meaning as that of $R^2$ in the formula (I). X represents a halogeno group.

The compound of the formula (I) can be prepared by condensation of the compound of the formula (2) and the compound represented by the formula (3) in the presence of an appropriate base.

(Reaction scheme 2)

A reaction scheme 2 is shown as an alternative method to the method of scheme 1.

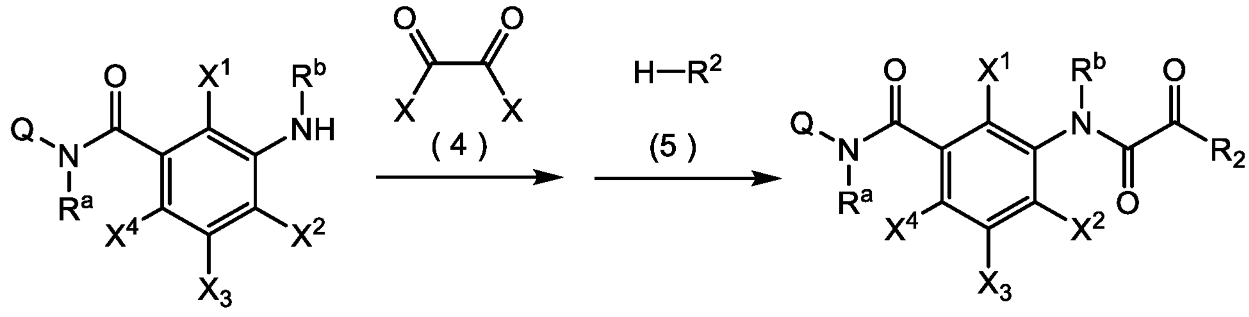


( 2 )　　　　( I )

The symbols in the formula (I) have the same meanings as those defined above. The symbols in the formula (2) have the same meanings as those defined in the formula (I). $R^2$ in the formula (5) has the same meaning as that of $R^2$ in the formula (I). X in the formula (4) represents a halogeno group.

The compound of the formula (I) can be prepared by condensation of the compound of the formula (2) and the compound represented by the formula (4), followed by reaction with the compound represented by the formula (5).

(Reaction scheme 3)

In addition to the above methods, a reaction scheme 3 is shown as an alternative method.

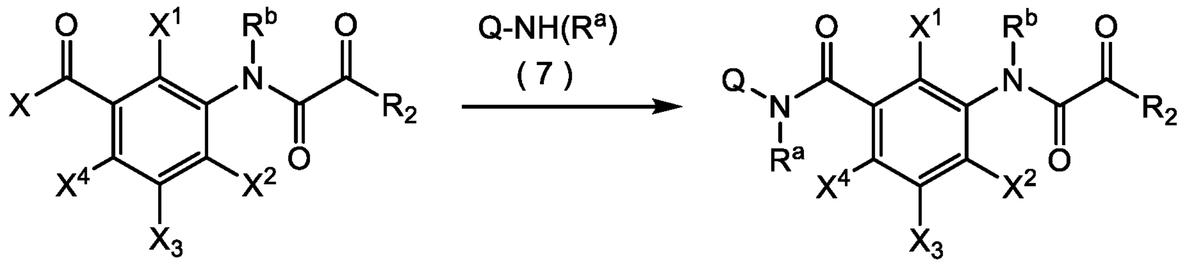


( 6 )　　　　( I )

The symbols in the formula (I) have the same meanings as those defined above. The symbols in the formula (6) have the same meanings as those defined in the formula (I), and X represents a halogeno group. Q and $R^a$ in the formula (7) have the same meanings as those defined in the formula (I).

The compound of the formula (I) can be prepared by condensation of the compound of the formula (6) and the compound represented by the formula (7) in the presence of an appropriate base.

The compound represented by the above formula (7) can be prepared with reference to a synthetic method described in Patent Document 1 (WO2017 / 102275A).

The compound represented by the formula (2) and the compound represented by the formula (6) can be prepared by using a compound represented by the following formula (8) as a raw material and employing various substituent conversions.

The symbols in the formula (8) have the same meanings as those defined in the formula (I), and R represents a lower alkyl group.

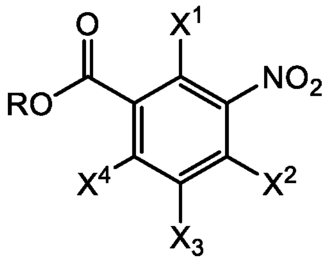

(8)

The benzamide compound of the present invention exhibits high herbicidal activity in both methods of soil treatment and foliage treatment under upland farming conditions.

The benzamide compound of the present invention is effective against various field weeds such as Digitaria ciliaris, Setaria faberi, Abutilon theophrasti, Amaranthus blitum, and Alopecurus myosuroides, and may show selectivity for crops such as maize and wheat.

In addition, the benzamide compound of the present invention may exhibit a plant growth regulating effect such as a growth inhibitory effect on useful plants such as crops, ornamental plants and fruit trees.

Moreover, the benzamide compound of the present invention has excellent herbicidal effects on paddy weeds such as Echinochloa spp., Cyperus difforis, Sagittaria trifolia and Schoenoplectiella hotarui, and may show selectivity for rice.

Furthermore, the benzamide compound of the present invention can also be applied for the control of weeds in places such as orchards, lawns, track ends and vacant sites.

Useful plants for which the herbicide of the present invention can be used include grains, for example, barley and wheat, and crops such as cotton, rapeseed, sunflower, maize, rice, soybean, sugar beet, sugar cane and lawn.

Crops may also include trees such as fruit trees, palm trees, coconut trees or other nuts, and also include vines such as grapes, fruit shrubs, fruit plants and vegetables.

Examples of upland field weeds to be controlled include the following weeds.

(A) Monocotyledonous weeds

(1) Weeds of the family Cyperaceae
Weeds of the genus Cyperus such as Cyperus esculentus, Cyperus iria, Cyperus microiria and Cyperus rotundus.
(2) Weeds of the family Poaceae

Weeds of the genus Alopecurus such as Alopecurus aequalis and Alopecurus myosuroides;
Weeds of the genus Apera such as Apera spica-venti;
Weeds of the genus Avena such as Avena sativa;
Weeds of the genus Bromus such as Bromus japonicus and Bromus sterilis;
Weeds of the genus Digitaria such as Digitaria ciliaris and Digitaria sanguinalis;
Weeds of the genus Echinochloa such as Echinochloa crus-galli;
Weeds of the genus Eleusine such as Eleusine indica;
Weeds of the genus Lolium such as Lolium multiflorum Lam.;
Weeds of the genus Panicum such as Panicum dichotomiflorum;
Weeds of the genus Poa such as Poa annua;
Weeds of the genus Setaria such as Setaria faberi, Setaria pumila and Setaria viridis;
Weeds of the genus Sorghum such as Sorghum bicolor; and
Weeds of the genus Urochloa such as Urochloa platyphylla.

(B) Dicotyledonous weeds

(1) Weeds of the family Amaranthaceae

Weeds of the genus Amaranthus such as Amaranthus blitum, Amaranthus palmeri, Amaranthus retroflexus and Amaranthus rudis;
Weeds of the genus Chenopodium such as Chenopodium album;
Weeds of the genus Bassia such as Bassia scoparia.

(2) Weeds of the family Asteraceae

Weeds of the genus Ambrosia such as Ambrosia artemisiifolia and Ambrosia trifida;
Weeds of the genus Conyza such as Conyza canadensis and Conyza sumatrensis;
Weeds of the genus Erigeron such as Erigeron annuus;
Weeds of the genus Matricaria such as Matricaria inodora and Matricaria recutita;
Weeds of the genus Xanthium such as Xanthium occidentale.

(3) Weeds of the family Caryophyllaceae

Weeds of the genus Sagina such as Sagina japonica;
Weeds of the genus Stellaria such as Stellaria media.

(4) Weeds of the family Convolvulaceae

Weeds of the genus Calystegia such as Calystegia japonica;
Weeds of the genus Ipomoea such as Ipomoea coccinea, Ipomoea hederacea, Ipomoea lacunosa and Ipomoea triloba.

(5) Weeds of the family Lamiaceae
Weeds of the genus Lamium such as Lamium album var. barbatum, Lamium amplexicaule and Lamium purpureum.
(6) Weeds of the family Malvaceae

Weeds of the genus Abutilon such as Abutilon theophrasti;
Weeds of the genus Sida such as Sida spinosa.

(7) Weeds of the family Plantaginaceae
Weeds of the genus Veronica such as Veronica persica.
(8) Weeds of the family Polygonaceae

Weeds of the genus Fallopia such as Fallopia convolvulus.
Weeds of the genus Persicaria such as Persicaria lapathifolia and Persicaria longiseta.

(9) Weeds of the family Rubiaceae
Weeds of the genus Galium, such as Galium spurium var. echinospermon.

Examples of paddy weeds to be controlled include the following weeds.

(A) Monocotyledonous weeds

(1) Weeds of the family Alismataceae
Weeds of the genus Sagittaria such as Sagittaria pygmaea Miq. and Sagittaria trifolia.
(2) Weeds of the family Cyperaceae

Weeds of the genus Cyperus such as Cyperus serotinus and Cyperus difforis;
Weeds of the genus Eleocharis such as Eleocharis kuroguwai Ohwi;
Weeds of the genus Schoenoplectiella such as Schoenoplectiella hotarui and Schoenoplectiella juncoides Roxb.
Weeds of the genus Scirpus such as Scirpus maritimus and Scirpus nipponicus.

(3) Weeds of the family Poaceae

Weeds of the genus Echinochloa such as Echinochloa oryzoides and Echinochloa crus-galli;
Weeds of the genus Leersia such as Leersia japonica;
Weeds of the genus Paspalum such as Paspalum distichum.

(4) Weeds of the family Pontederiaceae
Weeds of the genus Monochoria such as Monochoria korsakowii and Monochoria vaginalis var. plantaginea.

(B) Dicotyledonous weeds

(1) Weeds of the family Apiaceae
Weeds of the genus Oenanthe such as Oenanthe javanica.
(2) Weeds of the family Elatinaceae
Weeds of the genus Elatine such as Elatine triandra.
(3) Weeds of the family Linderniaceae
Weeds of the genus Lindernia such as Lindernia dubia subsp. major, Lindernia dubia subsp. dubia and Lindernia procumbens.
(4) Weeds of the family Lythraceae
Weeds of the genus Rotala such as Rotala indica var. uliginosa.

The herbicide of the present invention contains at least one selected from the group consisting of the benzamide compound of the present invention, that is, the compound (I) and a salt of the compound (I) as an active ingredient.

The herbicide of the present invention may consist only of the benzamide compound of the present invention, or may be formulated into a dosage form generally adopted as an agricultural chemical, for example, a wettable powder, a granule, a powder, an emulsion, a water soluble powder, a suspension, a flowable or the like.

A known additive or carrier can be used for formulation.

For solid dosage forms, vegetable powders such as soy flour and wheat flour, fine mineral powders such as diatomaceous earth, apatite, gypsum, talc, bentonite, pyrophyllite and clay, and organic and inorganic compounds such as sodium benzoate, urea and mirabilite can be used.

For liquid dosage forms, petroleum fractions such as kerosine, xylene and solvent naphtha, cyclohexane, cyclohexanone, dimethylformamide, dimethyl sulfoxide, alcohols, acetone, trichloroethylene, methyl isobutyl ketone, mineral oil, vegetable oil, water and the like can be used.

In formulation, a surfactant can be added as needed. Examples of the surfactant include nonionic surfactants such as alkylphenyl ethers to which polyoxyethylene is added, alkyl ethers to which polyoxyethylene is added, higher fatty acid esters to which polyoxyethylene is added, sorbitan higher fatty acid esters to which polyoxyethylene is added, and tristyrylphenyl ethers to which polyoxyethylene is added, sulfuric acid ester salts of alkylphenyl ethers to which polyoxyethylene is added, alkylnaphthalene sulfonates, polycarboxylates, lignin sulfonates, formaldehyde condensates of alkylnaphthalene sulfonates, and isobutylene-maleic anhydride copolymers.

In the herbicide of the present invention, the concentration of the active ingredient can be appropriately set according to the dosage form. For example, the concentration of the active ingredient in a wettable powder is preferably from 5 to 90% by weight, and more preferably from 10 to 85% by weight. The concentration of the active ingredient in an emulsion is preferably from 3 to 70% by weight, and more preferably from 5 to 60% by weight. The concentration of the active ingredient in a granule is preferably from 0.01 to 50% by weight, and more preferably from 0.05 to 40% by weight.

The wettable powder or emulsion obtained in this manner can be used as a suspension or emulsion by diluting with water to a predetermined concentration, and the granules can be directly sprayed on or mixed with the soil before or after germination of weeds. When applying the herbicide of the present invention to a farm field, an appropriate amount of 0.1 g or more of the active ingredient can be applied per hectare.

In addition, the herbicide of the present invention can also be used by mixing with a known fungicide, insecticide, acaricide, herbicide, plant growth regulator, fertilizer, phytotoxicity reducing agent (safener) or the like. In particular, it is possible to reduce the amount of drug used by using it in combination with a herbicide. Further, in addition to labor saving, even higher effects can also be expected due to the synergistic action of the mixed drug. In that case, a combination with a plurality of known herbicides is also possible.

Other herbicidal active ingredients used in the present invention are not particularly limited, and examples thereof include the following.

(a) aryloxyphenoxypropionic acid ester-based ingredients such as clodinafoppropargyl, cyhalofop-butyl, diclofop-methyl, fenoxaprop-P-ethyl, fluazifop-P, fluazifop-P-butyl, haloxyfop-methyl, pyriphenop-sodium, propaquizafop,

quizalofop-P-ethyl and metamifop; cyclohexanedione-based ingredients such as alloxydim, butroxydim, clethodim, cycloxydim, profoxydim, sethoxydim, tepraloxydim and tralkoxydim; phenylpyrazolin-based ingredients such as pinoxaden; and other ingredients that are said to exhibit herbicidal effects by inhibiting acetyl CoA carboxylase of plants.

(b) sulfonylurea-based ingredients such as amidosulfuron, azimsulfuron, bensulfuron-methyl, chlorimuron-ethyl, chlorsulfuron, cinosulfuron, cyclosulfamuron, ethametsulfuron-methyl, ethoxysulfuron, flazasulfuron, flupyrsulfuron, foramsulfuron, halosulfuron-methyl, imazosulfuron, iodosulfuron-methyl, mesosulfuron, mesosulfuronmethyl, metsulfuron-methyl, nicosulfuron, oxasulfuron, primisulfuron, prosulfuron, pyrazosulfuron-ethyl, rimsulfuron, sulfometuron-methyl, sulfosulfuron, thifensulfuronmethyl, triasulfuron, tribenuron-methyl, trifloxysulfuron, triflusulfuron-methyl, tritosulfuron, orthosulfamuron, propyrisulfuron, flucetosulfuron, metazosulfuron, methiopyrsulfuron, monosulfuron-methyl, orthosulfuron and iofensulfuron; imidazolinone-based ingredients such as imazapic, imazamethabenz, imazamoxammonium, imazapyr, imazaquin and imazethapyr; triazolopyrimidine sulfonamidebased ingredients such as cloransulam-methyl, diclosulam, florasulam, flumetsulam, metosulam, penoxsulam, pyroxsulam and metosulfam; pyrimidinyl(thio)benzoate-based ingredients such as bispyribac-sodium, pyribenzoxim, pyriftalid, pyrithiobac-sodium, pyriminobac-methyl and pyrimisulfan; sulfonyl amino carbonyl triazolinone-based ingredients such as flucarbazone, propoxycarbazone and thiencarbazone-methyl; sulfonanilide-based ingredients such as triafamone; and other ingredients that are said to exhibit herbicidal effects by inhibiting acetolactate synthase (ALS) (acetohydroxy acid synthase (AHAS)) of plants.

(c) triazine-based ingredients such as ametryn, atrazine, cyanazine, desmetryne, dimethametryn, prometon, prometryn, propazine-based ingredients (propazine), CAT (simazine), simetryn, terbumeton, terbuthylazine, terbutryne, trietazine, atratone and cybutryne; triazinone-based ingredients such as hexazinone, metamitron and metribuzin; triazolinone-based ingredients such as amicarbazone; uracil-based ingredients such as bromacil, lenacil and terbacil; pyridazinone-based ingredients such as PAC (chloridazon); carbamate-based ingredients such as desmedipham, phenmedipham and swep; urea-based ingredients such as chlorobromuron, chlorotoluron, chloroxuron, dimefuron, DCMU (diuron), ethidimuron, fenuron, fluometuron, isoproturon, isouron, linuron, methabenzthiazuron, metobromuron, metoxuron, monolinuron, neburon, siduron, tebuthiuron, metobenzuron and karbutilate; amide-based ingredients such as DCPA (propanil) and CMMP (pentanochlor); anilide-based ingredients such as cypromid; nitrile-based ingredients such as bromofenoxim, bromoxynil and ioxynil; benzothiadiazinone-based ingredients such as bentazone; phenylpyridazine-based ingredients such as pyridate and pyridafol; and other ingredients that are said to exhibit herbicidal effects by inhibiting photosynthesis of plants such as methazole.

(d) bipyridylium-based ingredients such as diquat and paraquat; and other ingredients that are said to become free radicals themselves in plants and generate active oxygen to exhibit fast-acting herbicidal effects.

(e) diphenyl ether-based ingredients such as acifluorfen-sodium, bifenox, chlomethoxynil (chlomethoxyfen), fluoroglycofen, fomesafen, halosafen, lactofen, oxyfluorfen, nitrofen and ethoxyfen-ethyl; phenylpyrazole-based ingredients such as fluazolate and pyraflufen-ethyl; N-phenylphthalimide-based ingredients such as cinidonethyl, flumioxazin, flumiclorac-pentyl and chlorphthalim; thiadiazole-based ingredients such as fluthiacet-methyl and thidiazimin; oxadiazole-based ingredients such as oxadiazon and oxadiargyl; triazolinone-based ingredients such as azafenidin, carfentrazone-ethyl, sulfentrazone and bencarbazone; oxazolidinedione-based ingredients such as pentoxazone; pyrimidinedione-based ingredients such as benzfendizone and butafenacil; sulfonylamide-based ingredients such as saflufenacil; pyridazine-based ingredients such as flufenpyr-ethyl; and other ingredients that are said to exhibit herbicidal effects by inhibiting chlorophyll biosynthesis in plants and abnormally accumulating photosensitizing peroxide substances in plant bodies, such as pyrachlonil, profluazol, tiafenacil and trifludimoxazin.

(f) pyridazinone-based ingredients such as norflurazon and metflurazon; pyridinecarboxamide-based ingredients such as diflufenican and picolinafen; triketonebased ingredients such as mesotrione, sulcotrione, tefuryltrione, tembotrione, bicyclopyrone and fenquinotrione; isoxazole-based ingredients such as isoxachlortole and isoxaflutole; pyrazole-based ingredients such as benzofenap, pyrazolynate, pyrazoxyfen, topramezone, pyrasulfotole and tolpyralate; triazole-based ingredients such as ATA (amitrol); isooxazolidinone-based ingredients such as clomazone; diphenyl ether-based ingredients such as aclonifen; and other ingredients that are said to exhibit herbicidal effects by inhibiting the biosynthesis of plant pigments such as carotenoids characterized by a bleaching action such as beflubutamid, fluridone, flurochloridone, flurtamone, benzobicyclone, methoxyphenone and ketospiradox.

(g) glycine-based ingredients such as glyphosate, glyphosate-ammonium, glyphosate-isopropylamine and glyphosate trimesium (sulfosate); and other ingredients inhibiting EPSP synthase

(h) phosphinic acid-based ingredients inhibiting glutamine synthetase such as glufosinate, glufosinate-ammonium and bialaphos (bilanafos), and other ingredients that are said to exhibit herbicidal effects by inhibiting the amino acid biosynthesis of plants.

(i) carbamate-based ingredients such as asulam; and other ingredients inhibiting DHP (dihydropteroate) synthase

(j) dinitroaniline-based ingredients such as bethrodine (benfluralin), butralin, dinitramine, ethalfluralin, oryzalin, pendimethalin, trifluralin, nitralin and prodiamine; phosphoroamidate-based ingredients such as amiprofos-methyl

and butamifos; pyridinebased ingredients such as dithiopyr and thiazopyr; benzamide-based ingredients such as propyzamide and tebutam; benzoic acid-based ingredients such as chlorthal and TCTP (chlorthal-dimethyl); carbamate-based ingredients such as IPC (chlorpropham), propham, carbetamide and barban; arylalanine-based ingredients such as flamprop-M and flamprop-M-isopropyl; chloroacetamide-based ingredients such as acetochlor, alachlor, butachlor, dimethachlor, dimethenamid, dimethenamid-P, metazachlor, metolachlor, Smetolachlor, pethoxamid, pretilachlor, propachlor, propisochlor and thenylchlor; acetamide-based ingredients such as diphenamid, napropamide and naproanilide; oxyacetamide-based ingredients such as flufenacet and mefenacet; tetrazolinone-based ingredients such as fentrazamide; and other ingredients that are said to exhibit herbicidal effects by inhibiting the microtubule polymerization, microtubule formation and cell division of plants or by inhibiting the biosynthesis of very long chain fatty acids (VLCFA), such as anilofos, indanofan, cafenstrole, piperophos, methiozolin, fenoxasulfone, pyroxasulfone and ipfencarbazone.

(k) nitrile-based ingredients such as DBN (dichlobenil) and DCBN (chlorthiamid); benzamide-based ingredients such as isoxaben; triazolocarboxamidebased ingredients such as flupoxam; quinoline carboxylic acid-based ingredients such as quinclorac; and other ingredients that are said to exhibit herbicidal effects by inhibiting the cell wall (cellulose) synthesis such as triaziflam and indaziflam.

(l) dinitrophenol-based ingredients such as DNOC, DNBP (dinoseb) and dinoterb; and other ingredients that are said to exhibit herbicidal effects by uncoupling (membrane disruption).

(m) thiocarbamate-based ingredients such as butylate, hexylthiocarbam (cycloate), dimepiperate, EPTC, esprocarb, molinate, orbencarb, pebulate, prosulfocarb, benthiocarb (thiobencarb), tiocarbazil, triallate, vernolate and diallate; phosphorodithioate-based ingredients such as SAP (bensulide); benzofuran-based ingredients such as benfuresate and ethofumesate; chlorocarbonic acid-based ingredients such as TCA, DPA (dalapon) and tetrapion (flupropanate); and other ingredients that are said to exhibit herbicidal effects by inhibiting the lipid biosynthesis of plants.

(n) phenoxycarboxylic acid-based ingredients such as clomeprop, 2,4-PA (2,4-D), 2,4-DB, dichlorprop, MCPA, MCPB and MCPP (mecoprop); benzoic acid-based ingredients such as chloramben, MDBA (dicamba) and TCBA (2,3,6-TBA); pyridinecarboxylic acid-based ingredients such as clopyralid, aminopyralid, fluroxypyr, picloram, triclopyr and halauxifen; quinoline carboxylic acid-based ingredients such as quinclorac and quinmerac; phthalamate semicarbazone-based ingredients such as NPA (naptalam) and diflufenzopyr; and other ingredients that are said to exhibit herbicidal effects by disturbing the hormone action of plants such as benazolin, diflufenzopyr, fluroxypyr, chlorflurenol, aminocyclopyrachlor, and DAS534.

(o) arylaminopropionic acid-based ingredients such as flamprop-isopropyl; pyrazolium-based ingredients such as difenzoquat; organic arsenic-based ingredients such as DSMA and MSMA; and other herbicides such as bromobutide, chlorflurenol, cinmethylin, cumyluron, dazomet, daimuron, methyl-dymron, etobenzanid, fosamine, oxaziclomefone, oleic acid, pelargonic acid, pyributicarb, endothall, sodium chlorate, metam, quinoclamine, cyclopyrimorate, tridiphane and clacyfos.

Examples of the safener that can be used in the present invention include benoxacor, cloquintocet, cloquintocet-mexyl, cyometrinil, cyprosulfamide, dichlormid, dicyclonon, dietholate, fenchlorazole, fenchlorazole-ethyl, fenclorim, flurazole, fluxofenim, furilazole, isoxadifen, isoxadifen-ethyl, mefenpyr, mefenpyr-diethyl, mephenate, naphthalic anhydride and oxabetrinil.

EXAMPLES

[Formulation Examples]

Although some formulation examples relating to the herbicide of the present invention are shown, the active ingredient compounds, additives and addition ratios are not limited only to those in the present examples and can be changed in a wide range. The term "part" in the formulation examples indicates "part by weight".

(Formulation Example 1) Wettable powder

| | |
|---|---|
| Compound of the present invention | 20 parts |
| White carbon | 20 parts |
| Diatomaceous earth | 52 parts |
| Sodium alkyl sulfate | 8 parts |

The above components are mixed uniformly and finely pulverized to obtain a wettable powder containing 20% of an active ingredient.

(Formulation Example 2) Emulsion

| | |
|---|---|
| Compound of the present invention | 20 parts |
| Xylene | 55 parts |
| Dimethylformamide | 15 parts |
| Polyoxyethylene phenyl ether | 10 parts |

The above components are mixed and dissolved to obtain an emulsion containing 20% of an active ingredient.

(Formulation Example 3) Granule

| | |
|---|---|
| Compound of the present invention | 5 parts |
| Talc | 40 parts |
| Clay | 38 parts |
| Bentonite | 10 parts |
| Sodium alkyl sulfate | 7 parts |

The above components are uniformly mixed and finely pulverized, and then granulated into a granular form having a diameter of 0.5 to 1.0 mm to obtain a granule containing 5% of an active ingredient.
Next, synthesis examples will be shown. However, the present invention is not limited to the following examples.

(Example 1)

$N^1$-(6-bromo-4-fluoro-2-methyl-3-((1-methyl-1H-tetrazol-5-yl)carbamoyl)phenyl)-$N^2$,$N^2$-dimethyloxalamide

Synthesis of [$N^1$-(6-bromo-4-fluoro-2-methyl-3-((1-methyl-1H-tetrazol-5-yl)carbamoyl)phenyl)-$N^2$,$N^2$-dimethyloxalamide]

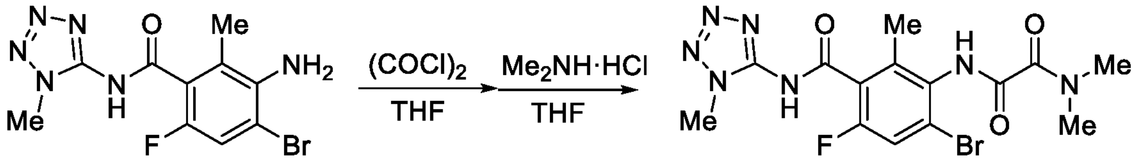


3-amino-4-bromo-6-fluoro-2-methyl-N-(1-methyl-1H-tetrazol-5-yl)benzamide (0.30 g, 0.91 mmol) was placed in a 50 mL flask, and then THF (15 mL) was added thereto, and the inside of the flask was purged with nitrogen. Oxalyl chloride (2.31 g, 18.22 mmol) was added thereto at room temperature, and the resulting mixture was stirred for 18 hours.
The solvent and excess oxalyl chloride were distilled off under reduced pressure, and THF (15 mL) was added. Dimethylamine-HCl (0.15 g, 1.82 mmol) and triethylamine (0.28 g, 2.73 mmol) were added thereto at 0°C. The resulting mixture was returned to room temperature and stirred under a nitrogen atmosphere at room temperature for 18 hours.
The obtained liquid was poured into a mixture of ethyl acetate (100 mL), water (100 mL) and 1N hydrochloric acid (50 mL), shaken, and separated. The organic layer was washed with brine (50 mL). Thereafter, the resultant was dried with magnesium sulfate. The obtained liquid was filtered, and the filtrate was concentrated. The concentrated product was purified by silica gel chromatography to obtain a desired product (0.17 g, yield: 45%).
Examples of the compounds of the present invention produced by the same method as in the above Examples are shown in Table 1. Physical property data of the compounds were entered in the column of "Physical properties". As

the physical property data, melting points (m.p.) were described. Table 2 shows $^1$H-NMR data for compounds marked with the symbol * in the column of "Physical properties".

In Table 1, "Me" indicates a methyl group and "Et" indicates an ethyl group. "J" indicates an iodo group.

Table 1

| Compound No. | Structural formula | Physical properties |
|---|---|---|
| A-1 | 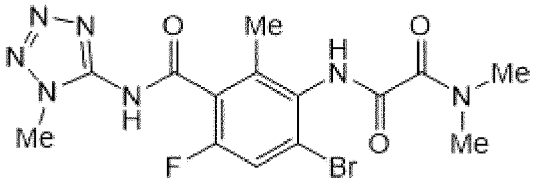 | * |
| A-2 | 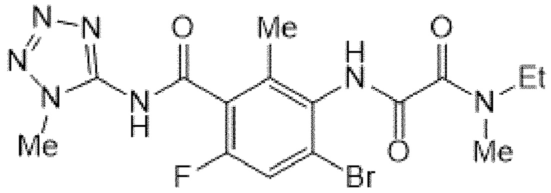 | m.p. 191-194°C |
| A-3 | 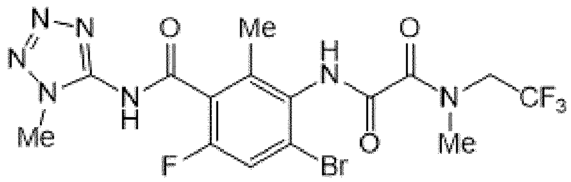 | * |
| A-4 | 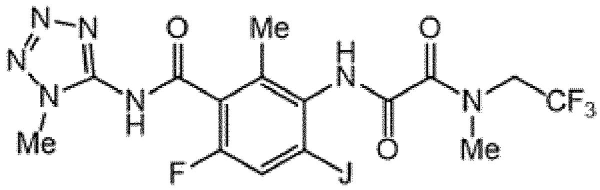 | m.p. 232-234°C |
| A-5 | 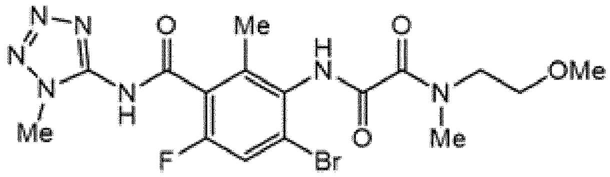 | m.p. 179-181°C |
| A-6 | 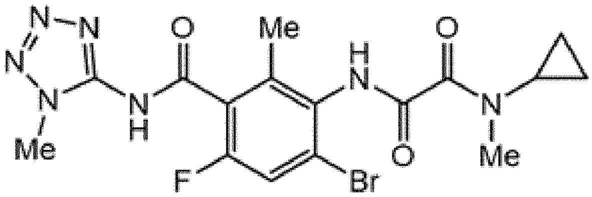 | m.p. 188-192°C |
| A-7 | 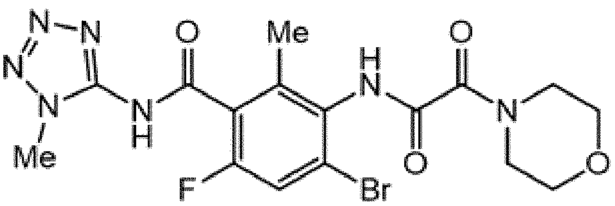 | m.p. 244-246°C |

(continued)

| Compound No. | Structural formula | Physical properties |
|---|---|---|
| A-8 | 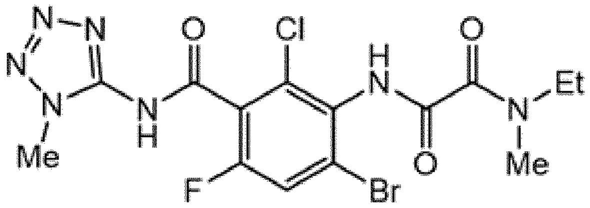 | m.p. 233-235°C |
| A-9 | 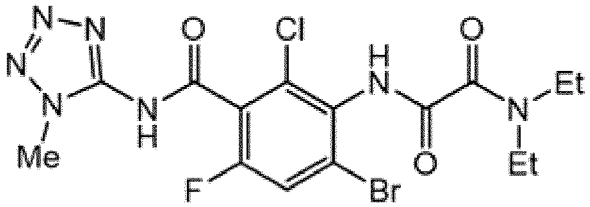 | m.p. 220-223°C |
| A-10 | 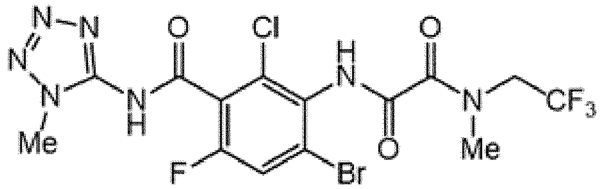 | m.p. 243-246°C |
| A-11 | 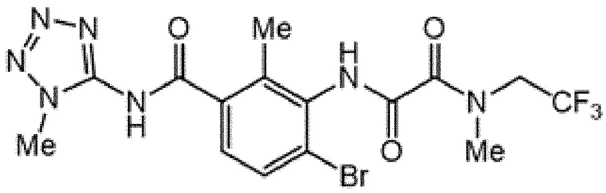 | m.p. 204-206°C |
| A-12 | 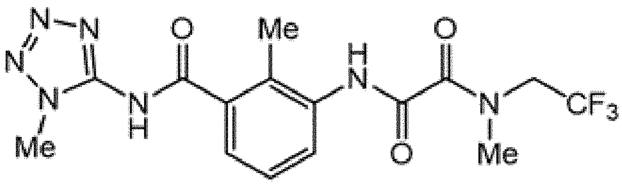 | m.p. 104-107°C |
| A-13 | 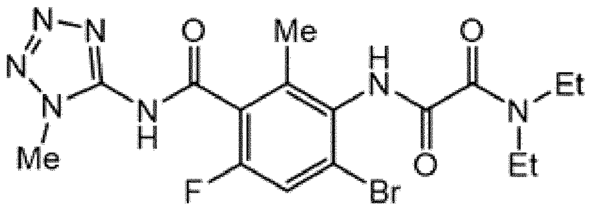 | m.p. 198-201°C |
| A-14 | 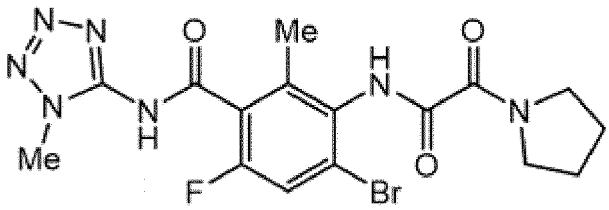 | m.p. 275-278°C |

(continued)

| Compound No. | Structural formula | Physical properties |
|---|---|---|
| A-15 | 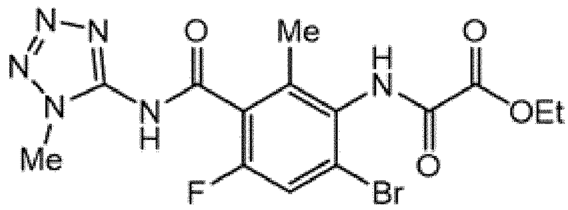 | m.p. 154-157°C |
| A-16 | 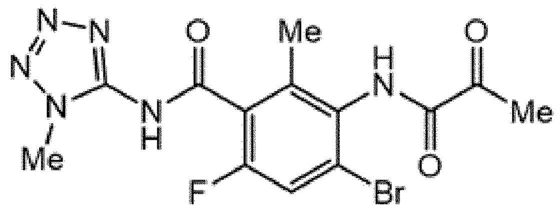 | m.p. 221-223°C |
| A-17 | 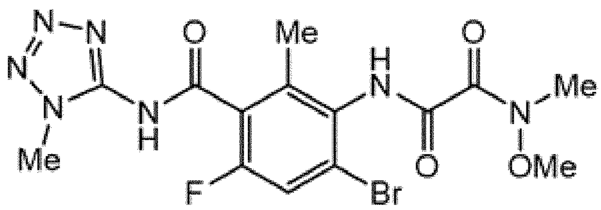 | m.p. 212-215°C |
| A-18 | 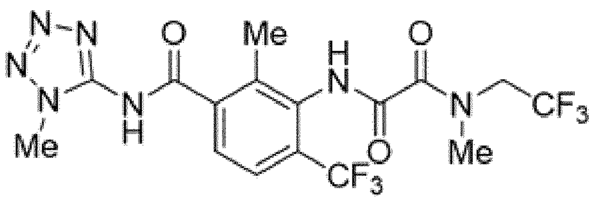 | m.p. 190-193°C |

Table 2

| Compound No. | 1H-NMR (400 MHz, $CDCl_3$) |
|---|---|
| A-1 | 2.35 (s, 3H), 3.08 (s, 3H), 3.41 (s, 3H), 4.10 (s, 3H), 7.37 (m, 1H), 9.01 (bs, 1H), 10.27 (bs, 1H). |
| A-3 | 2.31 (s, 3H), 3.19 (s, 3H), 4.07 (s, 3H), 4.74 (m, 2H), 7.37 (m, 1H), 9.09 (bs, 1H), 10.69 (bs, 1H). |

(Evaluation of herbicidal effects)

Next, the following test examples show that the benzamide compound of the present invention is useful as an active ingredient of a herbicide.

(Test Example 1)

POA allylphenyl ether (4.1 parts by weight), POE-POP glycol (1 part by weight), POE sorbitan laurate (0.8 parts by weight), glycerin (2.6 parts by weight), dimethylformamide (65.9 parts by weight), N-methylpyrrolidone (5.1 parts by weight), cyclohexanone (15.4 parts by weight), and aromatic hydrocarbons (5.1 parts by weight) were mixed to obtain an emulsion.

The compound of the present invention (4 mg) was dissolved in this emulsion (100 μL) to obtain a test emulsion. POA means "polyoxyalkylene", POE means "polyoxyethylene", and POP means "polyoxypropylene".

In a 150 $cm^2$ pot filled with soil, seeds of Digitaria ciliaris, Amaranthus retroflexus, Alopecurus myosuroides and Matricaria chamomilla were respectively sown and covered lightly with soil. Thereafter, they were grown in a greenhouse. When each plant grew to a plant height of 2 to 5 cm, the above test emulsion was diluted with water and sprayed

on the foliage with a small sprayer at a spray water volume of 250 L per hectare (the amount of active ingredient of 250 g per hectare).

After 3 weeks, the above ground weights of weeds in the untreated and treated areas were measured for each plant, and the weed killing rate was calculated by the following calculation formula. The results are shown in Table 3.

$$\text{Weed killing rate (\%)} = [\{(\text{above ground weight of weeds in untreated area}) - (\text{above ground weight of weeds in treated area})\} / (\text{above ground weight of weeds in untreated area})] \times 100$$

Table 3

| Compound No. | Amount of active ingredient (g/ha) | Weed killing rate | | | |
|---|---|---|---|---|---|
| | | *Digitaria ciliaris* | *Amaranthus retroflexus* | *Alopecurus myosuroides* | *Matricaria chamomilla* |
| A-1 | 250 | 100% | 100% | 100% | 100% |
| A-2 | 250 | 100% | 100% | 100% | 100% |
| A-3 | 250 | 100% | 100% | 100% | 100% |
| A-4 | 250 | 70% | 90% | 80% | 100% |
| A-5 | 250 | 80% | 80% | 70% | - |
| A-6 | 250 | 80% | 100% | 90% | 100% |
| A-7 | 250 | - | 70% | - | - |
| A-8 | 250 | 70% | 100% | 100% | 100% |
| A-9 | 250 | 70% | 80% | 100% | 100% |
| A-10 | 250 | - | 100% | 70% | 100% |
| A-11 | 250 | 100% | 100% | 100% | 100% |
| A-13 | 250 | 90% | 100% | 100% | 100% |
| A-14 | 250 | - | 80% | - | - |
| A-16 | 250 | - | 70% | - | 90% |

(Test Example 2)

In a 150 $cm^2$ pot filled with soil, seeds of Alopecurus myosuroides, Setaria faberi and Zea mays were sown and covered lightly with soil. Thereafter, they were grown in a greenhouse. When Alopecurus myosuroides and Setaria faberi grew to a plant height of 2 to 5 cm and Zea mays grew to 30 to 35 cm, the test emulsion obtained in Test Example 1 was diluted with water and sprayed on the foliage with a small sprayer at a spray water volume of 250 L per hectare (the amount of active ingredient of 250 g, 63 g, or 16 g per hectare).

Also for the compound represented by the formula (B) (Compound No. B-1), a test emulsion was obtained in the same manner as in Test Example 1, and Test Example 2 was carried out using the emulsion.

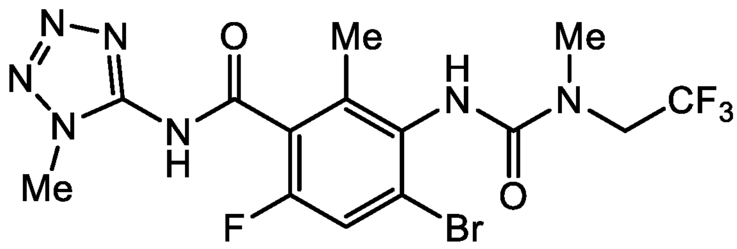

(B)

After 4 weeks, the above ground weights of weeds in the untreated and treated areas were measured for each plant, and the weed killing rate was calculated by the following calculation formula. The results are shown in Table 4.

$$\text{Weed killing rate (\%)} = [\{(\text{above ground weight of weeds in untreated area}) - (\text{above ground weight of weeds in treated area})\} / (\text{above ground weight of weeds in untreated area})] \times 100$$

Table 4

| Compound No. | Amount of active ingredient (g/ha) | Weed killing rate | | |
|---|---|---|---|---|
| | | *Alopecurus myosuroides* | *Setaria faberi* | *Zea mays* |
| A-3 | 250 | 100% | 100% | 30% |
| | 63 | 100% | 100% | 0% |
| | 16 | 80% | 50% | 0% |
| B-1 | 250 | 90% | 100% | 90% |
| | 63 | 50% | 50% | 30% |
| | 16 | 40% | 20% | 20% |

It can be seen that the compound of the present invention has dramatically improved herbicidal effects, and reduced phytotoxicity to maize, as compared with the conventional compounds.

Since all of those randomly selected from among the benzamide compounds of the present invention exert the above-mentioned effects, it can be understood that the benzamide compounds of the present invention including the compounds that are not exemplified are compounds having high herbicidal effects.

INDUSTRIAL APPLICABILITY

It is possible to provide a novel benzamide compound useful as an active ingredient of a herbicide, which has a reliable weed control effect even at a low dose, has less phytotoxicity to crops, and is highly safe for the environment; and a herbicide.

**Claims**

**1.** A compound represented by a formula (I) or a salt thereof:

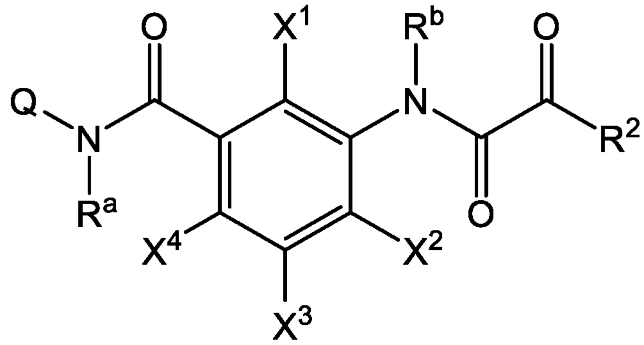

(I)

wherein

$R^a$ represents a hydrogen atom, a substituted or unsubstituted $C_{1-6}$ alkyl group, a substituted or unsubstituted $C_{1-6}$ alkylcarbonyl group, a substituted or unsubstituted $C_{1-6}$ alkoxycarbonyl group, or a substituted or unsubstituted $C_{6-10}$ arylcarbonyl group,
$R^b$ represents a hydrogen atom, a substituted or unsubstituted $C_{1-6}$ alkyl group, a substituted or unsubstituted $C_{1-6}$ alkylcarbonyl group, a substituted or unsubstituted $C_{1-6}$ alkoxycarbonyl group, or a substituted or unsubstituted $C_{6-10}$ arylcarbonyl group,
$X^1$ represents a hydrogen atom, a substituted or unsubstituted $C_{1-6}$ alkyl group, a substituted or unsubstituted $C_{1-6}$ alkylthio group, a substituted or unsubstituted $C_{1-6}$ alkylsulfinyl group, a substituted or unsubstituted $C_{1-6}$ alkylsulfonyl group, or a halogeno group,
$X^2$ represents a hydrogen atom, a substituted or unsubstituted $C_{1-6}$ alkyl group, a substituted or unsubstituted $C_{1-6}$ alkylthio group, a substituted or unsubstituted $C_{1-6}$ alkylsulfinyl group, a substituted or unsubstituted $C_{1-6}$ alkylsulfonyl group, or a halogeno group,
$X^3$ represents a hydrogen atom, a substituted or unsubstituted $C_{1-6}$ alkyl group, or a halogeno group,
$X^4$ represents a hydrogen atom or a halogeno group,
$R^2$ represents a substituted or unsubstituted $C_{1-6}$ alkyl group, a substituted or unsubstituted $C_{1-6}$ alkoxy group, or a group represented by a formula: $NR^{2a}R^{2b}$,
$R^{2a}$ represents a hydrogen atom, a substituted or unsubstituted $C_{1-6}$ alkyl group, or a substituted or unsubstituted $C_{1-6}$ alkoxy group,
$R^{2b}$ represents a hydrogen atom or a substituted or unsubstituted $C_{1-6}$ alkyl group,
$R^{2a}$ and $R^{2b}$ may form a divalent organic group together, and
Q represents a group represented by a formula (Q1), formula (Q2), formula (Q3) or formula (Q4),

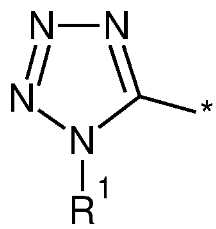

(Q1)

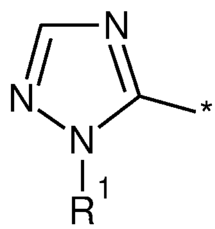

(Q2)

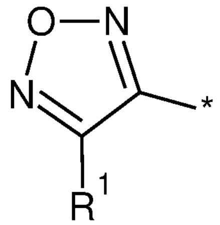

(Q3)

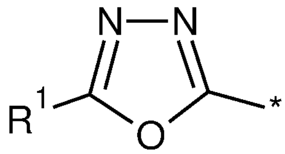

(Q4)

in the formula (Q1), formula (Q2), formula (Q3) and formula (Q4),
an asterisk (*) indicates a bonding position with an $R^a$-substituted nitrogen atom, and
each $R^1$ independently represents a hydrogen atom or a substituted or unsubstituted $C_{1-6}$ alkyl group.

**2.** The compound according to Claim 1 or a salt thereof,
wherein $R^2$ is a group represented by the formula: $NR^{2a}R^{2b}$ (the above $R^{2a}$ and $R^{2b}$ are as defined by the formula (I) in Claim 1.)

**3.** A herbicide comprising at least one selected from the group consisting of the compound according to either Claim 1 or 2 and a salt thereof as an active ingredient.

**INTERNATIONAL SEARCH REPORT**

International application No. PCT/JP2021/011323

A. CLASSIFICATION OF SUBJECT MATTER
Int. Cl. C07D257/06(2006.01)i, A01P13/00(2006.01)i, A01N43/713(2006.01)i, A01N43/84(2006.01)i
FI: C07D257/06 A CSP, A01P13/00, A01N43/713, A01N43/84 101
According to International Patent Classification (IPC) or to both national classification and IPC

B. FIELDS SEARCHED
Minimum documentation searched (classification system followed by classification symbols)
Int. Cl. C07D257/06, A01P13/00, A01N43/713, A01N43/84

Documentation searched other than minimum documentation to the extent that such documents are included in the fields searched
Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X | JP 60-042371 A (MERRELL DOW PHARMACEUTICALS INC.) 06 March 1985, examples 3-4, 5, first to fifth compounds | 1 |
| A | JP 2019-500365 A (BASF SE) 10 January 2019 | 1-3 |
| A | WO 2018/219935 A1 (BASF SE) 06 December 2018 | 1-3 |
| A | WO 2019/134993 A1 (BASF SE) 11 July 2019 | 1-3 |
| A | JP 9-509923 A (BAYER AG) 07 October 1997 | 1-3 |
| A | US 4406692 A (STAUFFER CHEMICAL COMPANY) 27 September 1983 | 1-3 |

☐ Further documents are listed in the continuation of Box C.   ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07.05.2021 | 18.05.2021 |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**
Information on patent family members

International application No.
PCT/JP2021/011323

| Patent Documents referred to in the Report | Publication Date | Patent Family | Publication Date |
|---|---|---|---|
| JP 60-042371 A | 06.03.1985 | US 4526979 A<br>examples 6-8<br>EP 135032 A1<br>NO 168641 B<br>NZ 208935 A<br>PT 78944 A<br>ES 534532 A<br>CA 1220209 A<br>DK 358584 A<br>HU 34461 A<br>IL 72431 A<br>ZA 8405518 A<br>AT 30024 T<br>IE 57604 B<br>GR 82102 A<br>KR 10-1989-0000534 B1<br>AU 3022284 A | |
| JP 2019-500365 A | 10.01.2019 | US 2018/0360046 A1<br>WO 2017/102275 A1<br>EP 3390372 A1<br>AR 107095 A<br>CA 3006838 A<br>AU 2016369900 A<br>IL 259627 D<br>CN 108473444 A<br>KR 10-2018-0095901 A<br>CR 20180370 A<br>MX 2018007418 A<br>BR 112018011776 A<br>CL 2018001559 A<br>EA 201891409 A<br>UA 121522 C | |
| WO 2018/219935 A1 | 06.12.2018 | US 2020/0157086 A1<br>EP 3630734 A1<br>CA 3063304 A<br>AU 2018275617 A | |
| WO 2019/134993 A1 | 11.07.2019 | EP 3508480 A1 | |
| JP 9-509923 A | 07.10.1997 | WO 1995/022532 A1<br>EP 746550 A1<br>AU 1808495 A<br>BR 9506928 A<br>HU 9602306 A<br>CA 2183641 A<br>HU 9602306 D<br>PL 315970 A<br>CN 1150421 A | |
| US 4406692 A | 27.09.1983 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2020051359 A
- WO 2017102275 A
- WO 2019105995 A